# EUROPÄISCHE PATENTSCHRIFT

(11) **EP 2 220 035 B1**
(45) Veröffentlichungstag und Bekanntmachung des Hinweises auf die Patenterteilung: **25.12.2019**
(21) Anmeldenummer: 08856412.5
(22) Anmeldetag: 02.12.2008
(51) Int. Cl.: C07C 275/60, C07C 273/18, C08G 18/62, C08G 18/75, C08G 18/78, C08G 18/79, C09D 175/04

(54) **ALLOPHANATGRUPPENHALTIGE POLYISOCYANATE**
POLYISOCYANATES CONTAINING ALLOPHANATE GROUPS
POLYISOCYANATES CONTENANT DES GROUPES ALLOPHANATE

(30) Priorität: 06.12.2007 EP 07122511
(43) Veröffentlichungstag der Anmeldung: 25.08.2010
(73) Patentinhaber: BASF SE, 67056 Ludwigshafen am Rhein (DE)
(72) Erfinder: MARTIN-PORTUGUES, Marta, 67071 Ludwigshafen (DE); JOKISCH, Carl, 68259 Mannheim (DE); SCHÄFER, Harald, 68219 Mannheim (DE); TUCHBREITER, Lydie, 67346 Speyer (DE); STEINBRECHER, Angelika Maria, 70193 Stuttgart (DE)
(74) Vertreter: BASF IP Association
(86) Internationale Anmeldenummer: PCT/EP2008/066612
(87) Internationale Veröffentlichungsnummer: WO 2009/071533

(56) Entgegenhaltungen:
- EP-A- 1 134 247
- EP-A- 1 721 920
- GB-A- 994 890

## Beschreibung

Die vorliegende Erfindung betrifft neue allophanatgruppenhaltige Polyisocyanate auf Basis von Isophorondiisocyanat und deren Verwendung.

GB 994890 beschreibt die Herstellung von Allophanaten aus Diisocyanaten und mono- oder polyfunktionellen Alkoholen mit Hilfe von Metallcarboxylaten, Metallchelaten und tertiären Aminen. Explizit offenbart wird die Umsetzung von Toluylendiisocyanat mit Trimethylolpropan und von Isophorondiisocyanat mit 1,6-Hexandiol.

EP 194 A1 beschreibt die Herstellung allophanatgruppenhaltigen Polyisocyanate von aliphatischen oder cycloaliphatischen Isocyanaten durch Katalyse von Säuren. Dazu wird bevorzugt von Urethanen von phenolischen Polyhydroxyverbindungen, ein- bis vierwertigen aliphatischen Alkoholen, ein- bis vierwertigen cycloaliphatischen Alkoholen, ein- bis vierwertigen araliphatischen Alkoholen oder höhermolekularen Polyolen ausgegangen. Als Diisocyanate für diese Umsetzung werden aliphatische oder cycloaliphatische Diisocyanate oder Xylylendiisocyanate angegeben.

Explizit offenbart Beispiel 8 der EP 194 A1 die Bildung eines allophanatgruppenhaltigen Polyisocyanats durch Umsetzung von Isophorondiisocyanat mit 1,6-Hexandiol und n-Butanol induziert durch Chlorwasserstoff.

Erfindungswesentlich ist gemäß der EP 194 A1 der Einsatz starker Protonensäuren als Katalysator.

Die aus der GB 994890 vorbekannten Metallcarboxylate, Metallchelate und tertiären Amine führen nach Angabe der EP 194 A1 zu einer beträchtlichen Bildung von dimeren und tertiären Polyisocyanaten neben der wunschgemäßen Allophanatbildung.

DE 102004015985 A1 beschreibt die Bildung von Allophanatpräpolymeren aus Isocyanaten und Polyetherpolyolen mit einem Molgewicht von 300 bis 20000 und einer Funktionalität von größer 1,9. Bevorzugte Isocyanate sind 1,6-Hexamethylendiisocyanat und/oder Isophorondiisocyanat und als Katalysatoren werden Lewis- oder Brønstedt-Säuren offenbart.

Explizit offenbart werden lediglich Produkte auf Basis 1,6-Hexamethylendiisocyanat mit Polyethern einer Funktionalität von 2.

DE 102004015982 A1beschreibt die Stabilisierung von Allophanatpräpolymeren aus Isocyanaten und Polyhydroxyverbindungen.

Explizit offenbart werden lediglich Produkte auf Basis 1,6-Hexamethylendiisocyanat mit Polyethern einer Funktionalität von 2.

DE 102004015983 A1 beschreibt die Herstellung von Allophanatpräpolymeren aus Isocyanaten und Polyhydroxyverbindungen einer mittleren Funktionalität von > 1,5 mit einem Zn-Katalysator.

Explizit offenbart werden lediglich Produkte auf Basis 1,6-Hexamethylendiisocyanat mit Polyethern einer Funktionalität von 2.

US 5290902 offenbart die Herstellung von Gemischen aus Allophanaten und Isocyanuraten im Verhältnis von 10:1 bis 1:10, wobei als Alkohole lediglich Monoalkohole beschrieben werden.

Trotz all dieser im Stand der Technik beschriebenen Verbindungen besteht weiterhin ein Bedarf an niedrigviskosen hochfunktionellen Polyisocyanaten.

Aufgabe der vorliegenden Erfindung war es, neue hochfunktionelle Polyisocyanate für Lacke, besonders für Klarlacke bereitzustellen, die eine hohe Kratzfestigkeit bei gleichzeitig guter Elastizität aufweisen. Zudem sollten die Produkte eine geringe Viskosität aufweisen, damit sie leichter in Lacke einarbeitbar sind.

Die Aufgabe wurde gelöst durch allophanatgruppenhaltige Polyisocyanate der Formel (I) 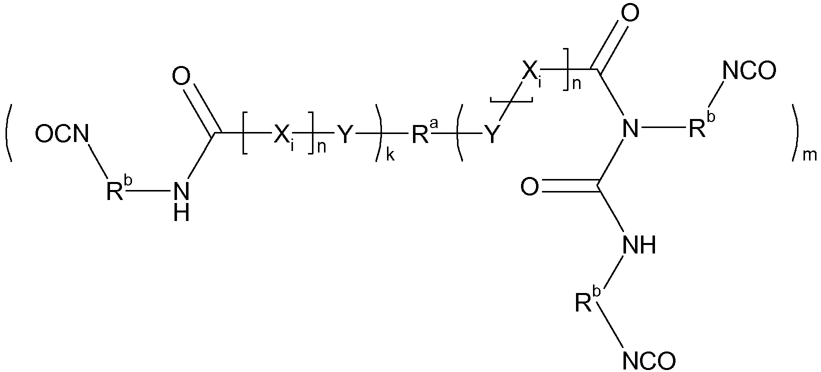 worin
R^{a} einen (k+m)-wertigen Rest, bevorzugt einen organischen Rest,
k 0 oder eine positive Zahl,
m eine positive Zahl,
mit der Maßgabe, daß (k + m) einen Wert von 3 bis 4 einnimmt,
Y für ein Sauerstoff- oder Stickstoffatom,
Xᵢ für jedes i von 1 bis n unabhängig voneinander ausgewählt ist aus der Gruppe bestehend aus -CH₂-CH₂-O-, -CH₂-CH(CH₃)-O-, -CH(CH₃)-CH₂-O-, -CH₂-C(CH₃)₂-O-,-C(CH₃)₂-CH₂-O-, -CH₂-CHVin-O-, -CHVin-CH₂-O-, -CH₂-CHPh-O- und -CHPh-CH₂-O-, bevorzugt aus der Gruppe -CH₂-CH₂-O-, -CH₂-CH(CH₃)-O- und -CH(CH₃)-CH₂-O-, und besonders bevorzugt -CH₂-CH₂-O-,
worin Ph für Phenyl und Vin für Vinyl steht,
n für jedes k und jedes m unabhängig voneinander 0 oder eine positive Zahl bedeutet, und
R^{b} für jedes k und jedes m unabhängig voneinander einen Rest 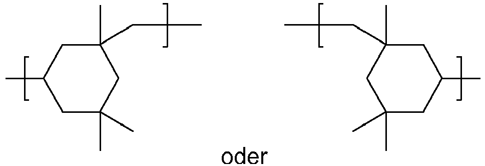 bedeutet.

Bei den den erfindungsgemäßen Allophanaten zugrundeliegenden Alkoholen handelt es sich um (k+m)-wertige Alkohole, die optional Gruppen Xᵢ tragen können, worin (k + m) erfindungsgemäß mindestens 3, bevorzugt 3 bis 6, besonders bevorzugt 3 bis 4 und ganz besonders bevorzugt 3 ist.

Die Werte für k, m und n können im statistischen Mittel ungeradzahlige Werte annehmen, sind dann aber natürlich bezogen auf jedes einzelne Molekül der Formel (I) geradzahlig.

Bevorzugt ist m > k, besonders bevorzugt is m ≥ (k+1), ganz besonders bevorzugt ist k ≤ 0,5, insbesondere ist k ≤ 0,2 und speziell ist k = 0.

Diese (k+m)-wertigen Alkohole weisen ein Molekulargewicht bevorzugt unter 500, besonders bevorzugt unter 400, ganz besonders bevorzugt unter 350, insbesondere unter 300 und speziell unter 250 g/mol auf.

Beispiele für derartige Alkohole R^{a}-(-Y-H)₍ₖ₊ₘ₎, in denen Y ein Sauerstoffatom darstellt, sind Trimethylolbutan, Trimethylolpropan, Trimethylolethan, Pentaerythrit, Glycerin, Ditrimethylolpropan, Dipentaerythrit, Sorbit, Mannit, Diglycerol, Threit, Erythrit, Adonit (Ribit), Arabit (Lyxit), Xylit oder Dulcit (Galactit).

Bevorzugt sind Trimethylolpropan, Pentaerythrit, Glycerin und Diglycerol, bevorzugt Trimethylolpropan und Glycerin und besonders bevorzugt Trimethylolpropan.

In einer Ausführungsform der vorliegenden Erfindung sind keine Gruppen Xᵢ in den Verbindungen der Formel (I) enthalten, d.h. n ist Null.

In einer weiteren Ausführungsform der vorliegenden Erfindung sind Gruppen Xᵢ anwesend und die Gesamtanzahl der Gruppen Xᵢ in der Verbindung der Formel (I), d.h. die Summe der Werte für n, beträgt bis zu sechzehn, bevorzugt drei bis zehn, besonders bevorzugt drei bis acht und ganz besonders bevorzugt drei bis sechs.

Gemäß dieser Ausführungsform handelt es sich um ethoxylierte und/oder propoxylierte Alkohole, bevorzugt entweder ausschließlich ethoxylierte oder ausschließlich propoxylierte Alkohole und besonders bevorzugt um ausschließlich ethoxylierte Alkohole.

Zur Erfüllung dieser Maßgabe kann die Zahl n für jedes k und jedes m unabhängig voneinander 0 oder eine positive Zahl bedeuten, beispielsweise 0 bis 10, bevorzugt 1 bis 5, besonders bevorzugt 1 bis 4, ganz besonders bevorzugt 1 bis 3 und insbesondere 1 bis 2.

Beispiele für Alkohole R^{a}-(-Y-[-Xᵢ]ₙ-H)₍ₖ₊ₘ₎, in denen Y ein Stickstoffatom darstellt, sind Triethanolamin, Tripropanolamin und 1,3,5,Tris-(2-hydroxyethyl)cyanursäure bevorzugt ist 1,3,5,Tris-(2-hydroxyethyl)cyanursäure. In diesen Fällen ist n jeweils 1 und Xᵢ jeweils -CH₂-CH₂-O-, -CH₂-CH(CH₃)-O- bzw. -CH(CH₃)-CH₂-O-.

Der Rest R^{b} ist abgeleitet von monomerem Isophorondiisocyanat, wobei es erfindungsgemäß eine untergeordnete Rolle spielt, ob die Urethan- bzw. Allophanatgruppe an ein primäres oder sekundäres Kohlenstoffatom gebunden ist. Je nach Reaktionsbedingungen (siehe unten) wird ein größerer Teil der Urethan- bzw. Allophanatgruppen an ein sekundäres Kohlenstoffatom gebunden sein. Gemäß E. Spyrou, Farbe und Lack 106, 10/2006, S. 126-130, kann man beispielsweise die Selektivität der Urethanreaktion durch unterschiedliche Katalysatoren beeinflussen.

Das zahlenmittlere Molekulargewicht Mₙ der allophanatgruppenhaltigen Polyisocyanate der Formel (I) beträgt in der Regel unter 2000, bevorzugt unter 1800, besonders bevorzugt unter 1500, ganz besonders bevorzugt unter 1200 und insbesondere unter 1100 g/mol.

Der NCO-Gehalt (berechnet als NCO mit eine Molgewicht von 42 g/mol) beträgt in der Regel mehr als 5 Gew%, bevorzugt mehr als 6 Gew% und besonders bevorzugt mehr als 8 Gew% und bis zu 17 Gew%, bevorzugt bis zu 15 Gew%.

Neben Allophanatgruppen können die erfindungsgemäßen allophanatgruppenhaltigen Polyisocyanate in untergeordneten Mengen noch weitere reaktive Gruppen tragen, beispielsweise unumgesetzte Hydroxygruppen sowie Isocyanuratgruppen.

Zur Herstellung der allophanatgruppenhaltigen Polyisocyanate werden Isophorondiisocyanat und der entsprechende alkoxylierte Alkohol mit oder ohne Lösungsmittel unter Urethanisierungsbedingungen und anschließend unter Allophanatisierungsbedingungen miteinander umgesetzt.

Die Temperatur beträgt bei dieser Umsetzung in der Regel bis zu 150 °C, bevorzugt bis zu 120 °C, besonders bevorzugt unter 100 °C und ganz besonders bevorzugt unter 90 °C und wird zumeist in Gegenwart mindestens eines Katalysators durchgeführt, der die Urethanisierungs- und/oder Allophanatiserungsreaktion katalysiert. Die Bildung der Urethangruppen kann aber auch in Abwesenheit eines Katalysators durchgeführt werden.

In der Regel sollte die Temperatur der Reaktion mindestens 20 °C betragen, bevorzugt mindestens 30, besonders bevorzugt mindestens 40 und ganz besonders bevorzugt mindestens 50 °C. In einer bevorzugten Ausführungsform beträgt die Reaktionstemperatur mindestens 80 °C.

Katalysatoren sind hierbei solche Verbindungen, die durch ihre Anwesenheit in einem Eduktgemisch zu einem höheren Anteil an urethan- bzw. allophanatgruppenhaltigen Reaktionsprodukten führen als das gleiche Eduktgemisch in deren Abwesenheit unter denselben Reaktionsbedingungen.

Diese sind beispielsweise organische Amine, insbesondere tertiäre aliphatische, cycloaliphatische oder aromatische Amine, und/oder Lewis-saure organische Metallverbindungen. Als Lewis-saure organische Metallverbindungen kommen z.B. Zinnverbindungen in Frage, wie beispielsweise Zinn-(II)-salze von organischen Carbonsäuren, z.B. Zinn-(II)-diacetat, Zinn-(II)-dioctoat, Zinn-(II)-bis(ethylhexanoat) und Zinn-(II)-dilaurat und die Dialkylzinn-(IV)-salze von organischen Carbonsäuren, z.B. Dimethylzinn-diacetat, Dibutylzinn-diacetat, Dibutylzinn-dibutyrat, Dibutylzinn-bis(2-ethylhexanoat), Dibutylzinn-dilaurat, Dibutylzinn-maleat, Dioctylzinn-dilaurat und Dioctylzinn-diacetat. Zudem können Zink-(II)-Salze eingesetzt werden, wie beispielsweise Zink-(II)-dioctoat. Auch Metallkomplexe wie Acetylacetonate des Eisens, Titans, Aluminiums, Zirkons, Mangans, Nickels, Zinks und Cobalts sind möglich. Weitere Metallkatalysatoren werden von Blank et al. in Progress in Organic Coatings, 1999, Vol. 35, Seiten 19-29 beschrieben.

Bevorzugte Lewis-saure organische Metallverbindungen sind Dimethylzinn-diacetat, Dibutylzinn-dibutyrat, Dibutylzinn-bis(2-ethylhexanoat), Dibutylzinn-dilaurat, Dioctylzinn-dilaurat, Zink-(II)-dioctoat, Zirkon-Acetylacetonat und Zirkon-2,2,6,6-tetramethyl-3,5-heptandionat.

Auch Wismut-und Cobaltkatalysatoren sowie Cäsiumsalze können als Katalysatoren eingesetzt werden. Als Cäsiumsalze kommen dabei solche Verbindungen in Betracht, in denen folgende Anionen eingesetzt werden: F⁻, Cl⁻, ClO⁻, ClO₃⁻, ClO₄⁻, Br⁻, J⁻, JO₃⁻, CN⁻, OCN⁻, NO₂⁻, NO₃⁻, HCO₃⁻, CO₃²⁻, S²⁻, SH⁻, HSO₃⁻, SO₃²⁻, HSO₄⁻, SO₄²⁻, S₂O₂²⁻, S₂O₄²⁻, S₂O₅²⁻, S₂O₆²⁻, S₂O₇²⁻, S₂O₈²⁻, H₂PO₂⁻, H₂PO₄⁻, HPO₄²⁻, PO₄³⁻, P₂O₇⁴⁻, (OCₙH₂ₙ₊₁)⁻, (CₙH₂ₙ₋₁O₂)⁻, (CₙH₂ₙ₋₃O₂)⁻ sowie (Cₙ₊₁H₂ₙ₋₂O₄)²⁻, wobei n für die Zahlen 1 bis 20 steht.

Bevorzugt sind dabei Cäsiumcarboxylate, bei denen das Anion den Formeln (CnH₂ₙ₋₁O₂)⁻ sowie (Cₙ₊₁H₂ₙ₋₂O₄)²⁻ mit n gleich 1 bis 20, gehorcht. Besonders bevorzugte Cäsiumsalze weisen als Anionen Monocarboxylate der allgemeinen Formel (CₙH₂ₙ₋₁O₂)⁻ auf, wobei n für die Zahlen 1 bis 20 steht. Hierbei sind insbesondere zu erwähnen Formiat, Acetat, Propionat, Hexanoat und 2-Ethylhexanoat.

Ferner sind als Katalysatoren einsetzbar:
- Organische Metallsalze der Formel (A)ₙ-R-O-CO-O^{⊖}M^{⊕} gemäß US-A-3 817 939, in der bedeuten:
   A eine Hydroxylgruppe oder ein Wasserstoffatom,
   n eine Zahl von 1 bis 3,
   R einen polyfunktionellen linearen oder verzweigten, aliphatischen oder aromatischen Kohlenwasserstoffrest und
   M^{⊕} in Kation, z.B. ein Alkalimetallkation oder ein quarternäres Ammoniumkation, wie Tetraalkylammonium, sowie
- quartäre Hydroxyalkylammoniumverbindungen der Formel
   R²⁴,R²⁵,R²⁶N^{⊕}-CH₂-CH(OH)-R^{27 ⊖}O-(CO)-R²⁸
   als Katalysator gemäß DE-A-26 31 733 (US-A-4 040 992) mit den dort angegeben Definitionen für die Reste.

Besonders geeignet als Katalysatoren für das Verfahren sind quartäre Ammoniumsalze entsprechend der Formel 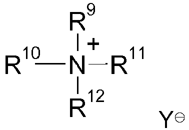 mit
Y^{⊖}= Carboxylat (R¹³COO⁻), Fluorid (F⁻), Carbonat (R¹³O(CO)O⁻) oder Hydroxid (OH⁻),
wie sie für Y⁻ = OH⁻ im US-Patent 4,324,879 und in den Deutschen Offenlegungsschriften 2,806,731 und 2,901,479 beschrieben sind.

Bevorzugt handelt es sich bei dem Rest Y^{⊖} um ein Carboxylat, Carbonat oder Hydroxid und besonders bevorzugt um ein Carboxylat oder Hydroxid.

R¹³ ist darin Wasserstoff, C₁ bis C₂₀-Alkyl, C₆ bis C₁₂-Aryl oder C₇ bis C₂₀-Arylalkyl, das jeweils optional substituiert sein kann.

Bevorzugt ist R¹³ Wasserstoff oder C₁ bis C₈-Alkyl.

Bevorzugte quartäre Ammoniumsalze sind diejenigen, bei denen die Reste R⁹ bis R¹² gleiche oder unterschiedliche Alkylgruppen mit 1 bis 20, vorzugsweise 1 bis 4, Kohlenstoffatomen darstellen, die gegebenenfalls durch Hydroxyl- oder Phenylgruppen substituiert sind.

Zwei der Reste R⁹ bis R¹² können auch zusammen mit dem Stickstoffatom und gegebenenfalls einem weiteren Stickstoff- oder Sauerstoffatom einen heterocyclischen, fünf-, sechs- oder siebengliedrigen Ring bilden. Die Reste R⁹ bis R¹¹ können in jedem Falle auch Ethylenreste darstellen, die zusammen mit dem quartären Stickstoffatom und einem weiteren tertiären Stickstoffatom eine bicyclische Triethylendiaminstruktur bilden, vorausgesetzt, daß der Rest R¹² dann eine Hydroxyalkylgruppe mit 2 bis 4 Kohlenstoffatomen darstellt, bei der die Hydroxylgruppe vorzugsweise in der 2-Stellung zu dem quartären Stickstoffatom angeordnet ist. Der hydroxysubstituierte Rest oder die hydroxysubstituierten Reste können auch andere Substituenten enthalten, beispielsweise C₁- bis C₄-Alkyloxy-Substituenten.

Dabei können die Ammoniumionen auch Teil eines ein- oder mehrgliedrigen Ringsystems sein, beispielsweise abgeleitet von Piperazin, Morpholin, Piperidin, Pyrrolidin, Chinuclidin oder 1,4-Di-aza-bicyclo-[2.2.2]-octan.

Beispiele für 1 bis 20 Kohlenstoffatome aufweisende Gruppen R⁹ bis R¹² sind unabhängig voneinander Methyl, Ethyl, Propyl, Isopropyl, n-Butyl, sec-Butyl, tert.-Butyl, Pentyl, Hexyl, Heptyl, Octyl, 2-Ethylhexyl, 2,4,4-Trimethylpentyl, Nonyl, Isononyl, Decyl, Dodecyl, Tetradecyl, Hetadecyl, Octadecyl, 1,1-Dimethylpropyl, 1,1-Dimethylbutyl, 1,1,3,3-Tetramethylbutyl, Benzyl, 1-Phenylethyl, 2-Phenylethyl, α,α-Dimethylbenzyl, Benzhydryl, p-Tolylmethyl,1-(p-Butylphenyl)-ethyl, p-Chlorbenzyl, 2,4-Dichlorbenzyl, p-Methoxybenzyl, m-Ethoxybenzyl, 2-Cyanoethyl, 2-Cyanopropyl, 2-Methoxycarbonethyl, 2-Ethoxycarbonylethyl, 2-Butoxycarbonylpropyl, 1,2-Di-(methoxycarbonyl)-ethyl, 2-Methoxyethyl, 2-Ethoxyethyl, 2-Butoxyethyl, Diethoxymethyl, Diethoxyethyl, Chlormethyl, 2-Chlorethyl, Trichlormethyl, Trifluormethyl, 1,1-Dimethyl-2-chlorethyl, 2-Methoxyisopropyl, 2-Ethoxyethyl, 2-Hydroxyethyl, 2-Hydroxypropyl, 3-Hydroxypropyl, 4-Hydroxybutyl, 6-Hydroxyhexyl, 2-Hydroxy-2,2-dimethylethyl, 2-Phenoxyethyl, 2-Phenoxypropyl, 3-Phenoxypropyl, 4-Phenoxybutyl, 6-Phenoxyhexyl, 2-Methoxyethyl, 2-Methoxypropyl, 3-Methoxypropyl, 4-Methoxybutyl, 6-Methoxyhexyl, 2-Ethoxyethyl, 2-Ethoxypropyl, 3-Ethoxypropyl, 4-Ethoxybutyl, 6-Ethoxyhexyl, Phenyl, Tolyl, Xylyl, α-Naphthyl, β-Naphthyl, 4-Diphenylyl, Chlorphenyl, Dichlorphenyl, Trichlorphenyl, Difluorphenyl, Methylphenyl, Dimethylphenyl, Trimethylphenyl, Ethylphenyl, Diethylphenyl, iso-Propylphenyl, tert.-Butylphenyl, Dodecylphenyl, Methoxyphenyl, Dimethoxyphenyl, Methylnaphthyl, Isopropylnaphthyl, Chlornaphthyl, 2,6-Dimethylphenyl, 2,4,6-Trimethylphenyl, 2,6-Dimethoxyphenyl, 2,6-Dichlorphenyl, Cyclopentyl, Cyclohexyl, Cyclooctyl, Cyclododecyl, Methylcyclopentyl, Dimethylcyclopentyl, Methylcyclohexyl, Dimethylcyclohexyl, Diethylcyclohexyl, Butylcyclohexyl, Chlorcyclohexyl, Dichlorcyclohexyl, Dichlorcyclopentyl, Norbornyl oder Norbornenyl.

Bevorzugte sind unabhängig voneinander die Reste R⁹ bis R¹² C₁ bis C₄-Alkyl. R¹² kann zusätzlich Benzyl sein oder ein Rest der Fomel 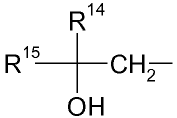 worin R¹⁴ und R¹⁵ unabhängig voneinander Wasserstoff oder C₁ bis C₄-Alkyl sein kann.

Besonders bevorzugte Reste R⁹ bis R¹² sind unabhängig voneinander Methyl, Ethyl und n-Butyl und für R¹² zusätzlich Benzyl, 2-Hydroxyethyl und 2-Hydroxypropyl.

Bevorzugt können für das erfindungsgemäße Verfahren folgende Katalysatoren eingesetzt werden:
Quarternäre Ammoniumhydroxide, vorzugsweise N,N,N-Trimethyl-N-benzylammoniumhydroxid und N,N,N-Trimethyl-N-(2-hydroxypropyl)-ammoniumhydroxid, gemäß DE-A-38 06 276.

Hydroxyalkyl substituierte quarternäre Ammoniumhydroxide gemäß EP-A-10 589 (US-A-4 324 879).

Organische Metallsalze der Formel (A)ₙ-R-O-CO-O^{⊖}M^{⊕} gemäß US-A-3 817 939, in der bedeuten, A eine Hydroxylgruppe oder ein Wasserstoffatom, n eine Zahl von 1 bis 3, R einen polyfunktionellen linearen oder verzweigten, aliphatischen oder aromatischen Kohlenwasserstoffrest und M ein Kation einer starken Base, z.B. ein Alkalimetallkation oder ein quarternäres Ammoniumkation, wie Tetraalkylammonium.

Bevorzugte Katalysatoren sind Zink-(II)-Salze, unter diesen besonders Zink Acetylacetonat.

Weiterhin bevorzugt sind die genannten Cäsium- und Wismutsalze.

Mit derartigen Katalysatoren ist es möglich, bei der Reaktion die auftretende Bildung von Polyisocyanaten zurückzudrängen. Somit ist es möglich, Reaktionsgemische zu erhalten, in denen das Verhältnis von Allophanatgruppen zu Isocyanuratgruppen mindestens 0,1:1, bevorzugt mindestens 0,3:1, besonders bevorzugt mindestens 0,5:1, ganz besonders bevorzugt mindestens 1:1, insbesondere mindestens 1,5:1, speziell mindestens 5:1 und sogar mindestens 10:1 beträgt.

Weiterhin bevorzugt ist Dibutylzinndilaurat.

Der Katalysator wird je nach Aktivität normalerweise in Mengen von 0,001 bis 10 mol% bzgl. eingesetzter Isocyanatgruppen eingesetzt, bevorzugt 0,5 bis 8, besonders bevorzugt 1 bis 7 und ganz besonders bevorzugt 2 bis 5 mol%.

Isophorondiisocyanat wird zumeist in mindestens doppelt äquimolarer Stöchiometrie bezogen auf die Hydroxygruppen im Alkohol eingesetzt, bevorzugt in einem 2,5 bis 10-fachem Überschuß von Isophorondiisocyanat zu Hydroxygruppen im Alkohol, bevorzugt im 3 bis 8- und besonders bevorzugt im 4 bis 5-fachen Überschuß.

Der unumgesetzte Teil Isophorondiisocyanat kann entweder im Reaktionsgemisch verbleiben oder wird bevorzugt abgetrennt, bevorzugt über eine Destillation, beispielsweise Kurzweg- oder Dünnschichtdestillation.

Der Gehalt an nicht umgesetztem Isophorondiisocyanat im Reaktionsgemisch beträgt in der Regel unter 1 Gew%, bevorzugt unter 0,5 Gew% und besonders bevorzugt unter 0,3 Gew%.

Die Reaktion wird bevorzugt ohne Lösungsmittel durchgeführt, kann aber auch in Gegenwart mindestens eines Lösungsmittel durchgeführt werden. Ebenso kann das erhaltene Reaktionsgemisch nach Beendigung der Reaktion in einem Lösungsmittel formuliert werden.

Als Lösungsmittel einsetzbar sind solche, die keine gegenüber Isocyanatgruppen reaktiven Gruppen aufweisen und in denen die Polyisocyanate zu mindestens 10 Gew%, bevorzugt zu mindestens 25, besonders bevorzugt zu mindestens 50, ganz besonders bevorzugt zu mindestens 75, insbesondere zu mindestens 90 und speziell zu mindestens 95 Gew% löslich sind.

Beispiele für derartige Lösungsmittel sind aromatische (einschließlich alkylierter Benzole und Naphthaline) und/oder (cyclo)aliphatische Kohlenwasserstoffe und deren Gemische, chlorierte Kohlenwasserstoffe, Ketone, Ester, alkoxylierte Alkansäurealkylester, Ether, respektive Gemische der Lösungsmittel.

Als aromatisch Kohlenwasserstoffgemische sind solche bevorzugt, die überwiegend aromatische C₇- bis C₁₄-Kohlenwasserstoffe umfassen und einen Siedebereich von 110 bis 300 °C umfassen können, besonders bevorzugt sind Toluol, o-, m- oder p-Xylol, Trimethylbenzolisomere, Tetramethylbenzolisomere, Ethylbenzol, Cumol, Tetrahydronaphthalin und solche enthaltende Gemische.

Beispiele dafür sind die Solvesso®-Marken der Firma ExxonMobil Chemical, besonders Solvesso® 100 (CAS-Nr. 64742-95-6, überwiegend C₉ und C₁₀-Aromaten, Siedebereich etwa 154 - 178 °C), 150 (Siedebereich etwa 182 - 207 °C) und 200 (CAS-Nr. 64742-94-5), sowie die Shellsol®-Marken der Firma Shell, Caromax® (z.B. Caromax® 18) der Firma Petrochem Carless und Hydrosol der Firma DHC (z.B. als Hydrosol® A 170). Kohlenwasserstoffgemische aus Paraffinen, Cycloparaffinen und Aromaten sind auch unter den Bezeichnungen Kristallöl (beispielsweise Kristallöl 30, Siedebereich etwa 158 - 198 °C oder Kristallöl 60: CAS-Nr. 64742-82-1), Testbenzin (beispielsweise ebenfalls CAS-Nr. 64742-82-1) oder Solventnaphtha (leicht: Siedebereich etwa 155 - 180 °C, schwer: Siedebereich etwa 225 - 300 °C) im Handel erhältlich. Der Aromatengehalt derartiger Kohlenwasserstoffgemische beträgt in der Regel mehr als 90 Gew%, bevorzugt mehr als 95, besonders bevorzugt mehr als 98 und ganz besonders bevorzugt mehr als 99 Gew%. Es kann sinnvoll sein, Kohlenwasserstoffgemische mit einem besonders verringerten Gehalt an Naphthalin einzusetzen.

(Cyclo)aliphatische Kohlenwasserstoffe sind beispielsweise Dekalin, alkyliertes Dekalin und Isomerengemische von geradlinigen oder verzweigten Alkanen und/oder Cycloalkanen.

Der Gehalt an aliphatischen Kohlenwasserstoffen beträgt in der Regel weniger als 5, bevorzugt weniger als 2,5 und besonders bevorzugt weniger als 1 Gew%.

Ester sind beispielsweise n-Butylacetat, Ethylacetat, 1-Methoxypropylacetat-2 und 2-Methoxyethylacetat.

Ether sind beispielsweise THF, Dioxan sowie die Dimethyl-, -ethyl- oder -n-butylether von Ethylenglykol, Diethylenglykol, Triethylenglykol, Propylenglykol, Dipropylenglykol oder Tripropylenglykol.

Ketone sind beispielsweise Aceton, Diethylketon, Ethylmethylketon, Isobutylmethylketon, Methylamylketon und tert-Butylmethylketon.

Die erfindungsgemäßen allophanatgruppenhaltigen Polyisocyanate finden beispielsweise Anwendung in zweikomponentigen Polyurethanlacken mit mindestens einer Komponente, die gegenüber Isocyanat reaktive Gruppen enthalten (Bindemittel). Dazu können sie allein oder im Gemisch mit anderen Polyisocyanaten als diesen erfindungsgemäßen allophanatgruppenhaltigen Polyisocyanaten als Vernetzerkomponente eingesetzt werden.

Derartige andere Polyisocyanate sind durch Oligomerisierung von monomeren Isocyanaten erhältlich.

Die eingesetzten monomeren Isocyanate können aromatisch, aliphatisch oder cycloaliphatisch sein, bevorzugt aliphatisch oder cycloaliphatisch, was in dieser Schrift kurz als (cyclo)aliphatisch bezeichnet wird, besonders bevorzugt sind aliphatische Isocyanate.

Aromatische Isocyanate sind solche, die mindestens ein aromatisches Ringsystem enthalten, also sowohl rein aromatische wie auch araliphatische Verbindungen.

Cycloaliphatische Isocyanate sind solche, die mindestens ein cycloaliphatisches Ringsystem enthalten.

Aliphatische Isocyanate sind solche, die ausschließlich gerade oder verzweigte Ketten enthalten, also acyclische Verbindungen.

Bei den monomeren Isocyanaten handelt es sich bevorzugt um Diisocyanate, die genau zwei Isocyanatgruppen tragen. Es kann sich aber prinzipiell auch um Monoisocyanate mit einer Isocyanatgruppe handeln.

Es kommen prinzipiell auch höhere Isocyanate mit im Mittel mehr als 2 Isocyanatgruppen in betracht. Dafür eignen sich beispielsweise Triisocyanate wie Triisocyanatononan, 2,4,6-Triisocyanatotoluol, Triphenylmethantriisocyanat oder 2,4,4'-Triisocyanatodiphenylether oder die Gemische aus Di-, Tri- und höheren Polyisocyanaten, die beispielsweise durch Phosgenierung von entsprechenden Anilin/Formaldehyd-Kondensaten erhalten werden und Methylenbrücken aufweisende Polyphenylpolyisocyanate darstellen.

Diese monomeren Isocyanate weisen keine wesentlichen Umsetzungsprodukte der Isocyanatgruppen mit sich selbst auf.

Bei den monomeren Isocyanaten handelt es sich bevorzugt um Isocyanate mit 4 bis 20 C-Atomen. Beispiele für übliche Diisocyanate sind aliphatische Diisocyanate wie Tetramethylendiisocyanat, 1,5-Pentamethylendiisocyanat, Hexamethylendiisocyanat (1,6-Diisocyanatohexan), Octamethylendiisocyanat, Decamethylendiisocyanat, Dodecamethylendiisocyanat, Tetradecamethylendiisocyanat, Derivate des Lysindiisocyanates, Trimethylhexandiisocyanat oder Tetramethylhexandiisocyanat, cycloaliphatische Diisocyanate wie 1,4-, 1,3- oder 1,2-Diisocyanatocyclohexan, 4,4'- oder 2,4'-Di(isocyanatocyclohexyl)methan, 1-lsocyanato-3,3,5-trimethyl-5-(isocyanatomethyl)cyclohexan (Isophorondiisocyanat), 1,3- oder 1,4-Bis(isocyanatomethyl)cyclohexan oder 2,4-, oder 2,6-Diisocyanato-1-methylcyclohexan sowie 3 (bzw. 4), 8 (bzw. 9)-Bis(isocyanatomethyl)-tricyclo[5.2.1.0^{2.6}]decan-Isomerengemische, sowie aromatische Diisocyanate wie 2,4- oder 2,6-Toluylendiisocyanat und deren Isomerengemische, m- oder p-Xylylendiisocyanat, 2,4'- oder 4,4'-Diisocyanatodiphenylmethan und deren Isomerengemische, 1,3- oder 1,4-Phenylendiisocyanat, 1-Chlor-2,4-phenylendiisocyanat, 1,5-Naphthylendiisocyanat, Diphenylen-4,4'-diisocyanat, 4,4'-Diisocyanato-3,3'-dimethyldiphenyl, 3-Methyldiphenylmethan-4,4'-diisocyanat, Tetramethylxylylendiisocyanat, 1,4-Diisocyanatobenzol oder Diphenylether-4,4'-diisocyanat.

Besonders bevorzugt sind 1,6-Hexamethylendiisocyanat, 1,3-Bis(isocyanatomethyl)cyclo-hexan, Isophorondiisocyanat und 4,4'- oder 2,4'-Di(isocyanatocyclohexyl)methan, ganz besonders bevorzugt sind Isophorondiisocyanat und 1,6-Hexamethylendiisocyanat, insbesondere bevorzugt ist 1,6-Hexamethylendiisocyanat.

Es können auch Gemische der genannten Isocyanate vorliegen.

Isophorondiisocyanat liegt zumeist als ein Gemisch, und zwar der cis- und trans-Isomere vor, in der Regel im Verhältnis von ca. 60:40 bis 80:20 (w/w), bevorzugt im Verhältnis von ca. 70:30 bis 75:25 und besonders bevorzugt im Verhältnis von ca. 75:25.

Dicyclohexylmethan-4,4'-diisocyanat kann ebenfalls als Gemisch der verschiedenen cis- und trans-Isomere vorliegen.

Für die vorliegende Erfindung können sowohl solche Diisocyanate eingesetzt werden, die durch Phosgenierung der korrespondierenden Amine erhalten werden, als auch solche, die ohne die Verwendung von Phosgen, d. h. nach phosgenfreien Verfahren, hergestellt werden. Nach Angaben der EP-A-0 126 299 (US 4 596 678), EP-A-126 300 (US 4 596 679) und EP-A-355 443 (US 5 087 739) beispielsweise können (cyclo)aliphatische Diisocyanate, z.B. wie 1,6-Hexamethylendiisocyanat (HDI), isomere aliphatische Diisocyanate mit 6 Kohlenstoffatomen im Alkylenrest, 4,4'- oder 2,4'-Di(isocyanatocyclohexyl)methan und 1-Isocyanato-3-isocyanato-methyl-3,5,5-trimethylcyclohexan (Isophorondiisocyanat bzw. IPDI) hergestellt werden durch Umsetzung der (cyclo)aliphatischen Diamine mit beispielsweise Harnstoff und Alkoholen zu (cyclo)aliphatischen Biscarbaminsäureestern und deren thermische Spaltung in die entsprechenden Diisocyanate und Alkohole. Die Synthese erfolgt meist kontinuierlich in einem Kreislaufverfahren und gegebenenfalls in Gegenwart von N-unsubstituierten Carbaminsäureestern, Dialkylcarbonaten und anderen aus dem Reaktionsprozeß zurückgeführten Nebenprodukten. So erhaltene Diisocyanate weisen in der Regel einen sehr geringen oder sogar nicht meßbaren Anteil an chlorierten Verbindungen auf, was beispielsweise in Anwendungen in der Elektronikindustrie vorteilhaft ist.

In einer Ausführungsform der vorliegenden Erfindung weisen die eingesetzten Isocyanate einen Gesamtgehalt an hydrolysierbarem Chlor von weniger als 200 ppm auf, bevorzugt von weniger als 120 ppm, besonders bevorzugt weniger als 80 ppm, ganz besonders bevorzugt weniger als 50 ppm, insbesondere weniger als 15 ppm und speziell weniger als 10 ppm. Dies kann beispielsweise gemessen werden durch die ASTM-Vorschrift D4663-98. Es können aber selbstverständlich auch monomere Isocyanate mit einem höheren Chlorgehalt eingesetzt werden, beispielsweise bis zu 500 ppm.

Selbstverständlich können auch Gemische aus solchen monomeren Isocyanaten, die durch Umsetzung der (cyclo)aliphatischen Diamine mit beispielsweise Harnstoff und Alkoholen und Spaltung der erhaltenen (cyclo)aliphatischen Biscarbaminsäureester erhalten worden sind, mit solchen Diisocyanaten, die durch Phosgenierung der korrespondierenden Amine erhalten worden sind, eingesetzt werden.

Die Polyisocyanate, zu denen die monomeren Isocyanate oligomerisiert werden können, sind in der Regel wie folgt charakterisiert:
Die mittlere NCO Funktionalität solcher Verbindungen beträgt in der Regel mindestens 1,8 und kann bis zu 8 betragen, bevorzugt 2 bis 5 und besonders bevorzugt 2,4 bis 4.

Der Gehalt an Isocyanatgruppen nach der Oligomerisierung, berechnet als NCO = 42 g/mol, beträgt, wenn nicht anders angegeben, in der Regel von 5 bis 25 Gew%.

Bevorzugt handelt es sich bei den anderen Polyisocyanaten als den erfindungsgemäßen allophanatgruppenhaltigen Polyisocyanaten um folgende Verbindungen:
1) Isocyanuratgruppen aufweisende Polyisocyanate von aromatischen, aliphatischen und/oder cycloaliphatischen Diisocyanaten. Besonders bevorzugt sind hierbei die entsprechenden aliphatischen und/oder cycloaliphatischen Isocyanato-Isocyanurate und insbesondere die auf Basis von Hexamethylendiisocyanat und Isophorondiisocyanat. Bei den dabei vorliegenden Isocyanuraten handelt es sich insbesondere um Tris-isocyanatoalkyl- bzw. Tris-isocyanatocycloalkyl-Isocyanurate, welche cyclische Trimere der Diisocyanate darstellen, oder um Gemische mit ihren höheren, mehr als einen Isocyanuratring aufweisenden Homologen. Die Isocyanato-Isocyanurate haben im allgemeinen einen NCO-Gehalt von 10 bis 30 Gew.-%, insbesondere 15 bis 25 Gew.-% und eine mittlere NCO-Funktionalität von 2,6 bis 8.
2) Uretdiongruppen aufweisende Polyisocyanate mit aromatisch, aliphatisch und/oder cycloaliphatisch gebundenen Isocyanatgruppen, vorzugsweise aliphatisch und/oder cycloaliphatisch gebundenen und insbesondere die von Hexamethylendiisocyanat oder Isophorondiisocyanat abgeleiteten. Bei Uretdiondiisocyanaten handelt es sich um cyclische Dimerisierungsprodukte von Diisocyanaten.
   Die Uretdiongruppen aufweisenden Polyisocyanate werden im Rahmen dieser Erfindung im Gemisch mit anderen Polyisocyanaten, insbesondere den unter 1) genannten, erhalten. Dazu können die Diisocyanate unter Reaktionsbedingungen umgesetzt werden, unter denen sowohl Uretdiongruppen als auch die anderen Polyisocyanate gebildet werden, oder zunächst die Uretdiongruppen gebildet und diese anschließend zu den anderen Polyisocyanaten umgesetzt werden oder die Diisocyanate zunächst zu den andereren Polyisocyanaten und diese anschließend zu Uretdiongruppen-haltigen Produkten umgesetzt werden.
3) Biuretgruppen aufweisende Polyisocyanate mit aromatisch, cycloaliphatisch oder aliphatisch gebundenen, bevorzugt cycloaliphatisch oder aliphatisch gebundenen Isocyanatgruppen, insbesondere Tris-(6-isocyanatohexyl)-biuret oder dessen Gemische mit seinen höheren Homologen. Diese Biuretgruppen aufweisenden Polyisocyanate weisen im allgemeinen einen NCO-Gehalt von 18 bis 22 Gew.-% und eine mittlere NCO-Funktionalität von 2,8 bis 6 auf.
4) Urethan- und/oder Allophanatgruppen aufweisende Polyisocyanate mit aromatisch, aliphatisch oder cycloaliphatisch gebundenen, bevorzugt aliphatisch oder cycloaliphatisch gebundenen Isocyanatgruppen, wie sie beispielsweise durch Umsetzung von überschüssigen Mengen an Diisocyanat, beispielsweise Hexamethylendiisocyanat oder Isophorondiisocyanat, mit ein- oder mehrwertigen Alkoholen (A). Diese Urethan- und/oder Allophanatgruppen aufweisenden Polyisocyanate haben im allgemeinen einen NCO-Gehalt von 12 bis 24 Gew.-% und eine mittlere NCO-Funktionalität von 2,5 bis 4,5. Solche Urethan- und/oder Allophanatgruppen aufweisenden Polyisocyanate können unkatalysiert oder bevorzugt in Gegenwart von Katalysatoren, wie beispielsweise Ammoniumcarboxylaten oder -hydroxiden, oder Allophanatisierungskatalysatoren, z.B. Zn-(II)-Verbindungen, jeweils in Anwesenheit von ein-, zwei- oder mehrwertigen, bevorzugt einwertigen Alkoholen, hergestellt werden.
5) Oxadiazintriongruppen enthaltende Polyisocyanate, vorzugsweise von Hexamethylendiisocyanat oder Isophorondiisocyanat abgeleitet. Solche Oxadiazintriongruppen enthaltenden Polyisocyanate sind aus Diisocyanat und Kohlendioxid zugänglich.
6) Iminooxadiazindiongruppen enthaltende Polyisocyanate, vorzugsweise von Hexamethylendiisocyanat oder Isophorondiisocyanat abgeleitet. Solche Iminooxadiazin-diongruppen enthaltenden Polyisocyanate sind aus Diisocyanaten mittels spezieller Katalysatoren herstellbar.
7) Uretonimin-modifizierte Polyisocyanate.
8) Carbodiimid-modifizierte Polyisocyanate.
9) Hyperverzweigte Polyisocyanate, wie sie beispielsweise bekannt sind aus der DE-A1 10013186 oder DE-A1 10013187.
10) Polyurethan-Polyisocyanat-Präpolymere, aus Di- und/oder Polyisocyanaten mit Alkoholen.
11) Polyharnstoff-Polyisocyanat-Präpolymere.
12) Die Polyisocyanate 1)-11), bevorzugt 1), 3), 4) und 6) können nach deren Herstellung in Biuretgruppen- oder Urethan-/Allophanat-Gruppen aufweisende Polyisocyanate mit aromatisch, cycloaliphatisch oder aliphatisch gebundenen, bevorzugt (cyclo)aliphatisch gebundenen Isocyanatgruppen, überführt werden. Die Bildung von Biuretgruppen erfolgt beispielsweise durch Zugabe von Wasser oder Umsetzung mit Aminen. Die Bildung von Urethan- und/oder Allophanatgruppen erfolgt durch Umsetzung mit ein-, zwei- oder mehrwertigen, bevorzugt einwertigen Alkoholen, gegebenenfalls in Gegenwart von geeigneten Katalysatoren. Diese Biuret- oder Urethan-/Allophanatgruppen aufweisenden Polyisocyanate weisen im allgemeinen einen NCO-Gehalt von 18 bis 22 Gew.-% und eine mittlere NCO-Funktionalität von 2,8 bis 6 auf.
13) Hydrophil modifizierte Polyisocyanate, d.h. Polyisocyanate, die neben den unter 1-12 beschriebenen Gruppen solche enthalten, die formal durch Addition von Molekülen mit NCO-reaktiven Gruppen und hydrophilierenden Gruppen an die Isocyanatgruppen obiger Moleküle entstehen. Bei letzteren handelt es sich um nichtionische Gruppen wie Alkyl-Polyethylenoxid und/oder ionische, welche von Phosphorsäure, Phosphonsäure, Schwefelsäure oder Sulfonsäure, bzw. ihren Salzen abgeleitet sind.
14) Modifizierte Polyisocyanate für Dual Cure Anwendungen, d.h. Polyisocyanate, die neben den unter 1-12 beschriebenen Gruppen solche enthalten, die formal durch Addition von Molekülen mit NCO-reaktiven Gruppen und durch UV- oder aktinische Strahlung vernetzbare Gruppen an die Isocyanatgruppen obiger Moleküle entstehen. Bei diesen Moleküle handelt es sich beispielsweise um Hydroxyalkyl(meth)acrylate und andere Hydroxy-Vinylverbindungen.

Die oben aufgeführten Diisocyanate oder Polyisocyanate können auch zumindest teilweise in blockierter Form vorliegen.

Zur Blockierung eingesetzte Verbindungsklassen sind beschrieben in D. A. Wicks, Z. W. Wicks, Progress in Organic Coatings, 36, 148-172 (1999), 41, 1-83 (2001) sowie 43, 131-140 (2001).

Beispiele für zur Blockierung eingesetzte Verbindungsklassen sind Phenole, Imidazole, Triazole, Pyrazole, Oxime, N-Hydroxyimide, Hydroxybenzoesäureester, sekundäre Amine, Lactame, CH-acide cyclische Ketone, Malonsäureester oder Alkylacetoacetate.

In einer bevorzugten Ausführungsform der vorliegenden Erfindung ist das Polyisocyanat ausgewählt aus der Gruppe bestehend aus Isocyanuraten, Biureten, Urethanen und Allophanaten, bevorzugt aus der Gruppe bestehend aus Isocyanuraten, Urethanen und Allophanaten, besonders bevorzugt aus der Gruppe bestehend aus Isocyanuraten und Allophanaten, insbesondere handelt es sich um ein isocyanuratgruppenhaltiges Polyisocyanat.

In einer besonders bevorzugten Ausführungsform handelt es sich bei dem Polyisocyanat um Isocyanuratgruppen enthaltende Polyisocyanate von 1,6-Hexamethylendiisocyanat und/oder Isophorondiisocyanat.

In einer weiteren besonders bevorzugten Ausführungsform handelt es sich bei dem Polyisocyanat um ein Gemisch von Isocyanuratgruppen enthaltenden Polyisocyanaten von 1,6-Hexamethylendiisocyanat und von Isophorondiisocyanat.

In einer besonders bevorzugten Ausführungsform handelt es sich bei dem Polyisocyanat um ein Gemisch enthaltend niedrigviskose Polyisocyanate, bevorzugt Isocyanuratgruppen enthaltende Polyisocyanate, mit einer Viskosität von 600-1500 mPa*s, insbesondere unter 1200 mPa*s, niederviskose Urethane und/oder Allophanate mit einer Viskosität von 200-1600 mPa*s, insbesondere 600-1500 mPa*s, und/oder Iminooxadiazindiongruppen enthaltende Polyisocyanate.

In dieser Schrift wird die Viskosität bei 23 °C gemäß DIN EN ISO 3219/A.3 in einem Kegel-Platte-System mit einem Geschwindigkeitsgefälle von 1000 s⁻¹ angegeben, falls nicht anders vermerkt.

Die erfindungsgemäßen allophanatgruppenhaltigen Polyisocyanate können gegebenenfalls im Gemisch mit anderen Polyisocyanat als Vernetzerkomponenten mit mindestens einem Bindemittel in Polyurethanlacken eingesetzt werden.

In der Regel werden für Polyisocyanatzusammensetzungen, also die Summe der isocyanatgruppenhaltigen Verbindungen,
50 bis 100 Gew% der erfindungsgemäßen allophanatgruppenhaltigen Polyisocyanate eingesetzt, bevorzugt 50 bis 90 Gew% und besonders bevorzugt 60 bis 80 Gew%, und 0 bis 50 Gew% andere Polyisocyanate, bevorzugt 10 bis 50, besonders bevorzugt 20 bis 40 Gew%,
mit der Maßgabe, daß die Summe immer 100 Gew% beträgt.

Bei den Bindemitteln kann es sich beispielsweise um Polyacrylatpolyole, Polyesterpolyole, Polyetherpolyole, Polyurethanpolyole; Polyharnstoffpolyole; Polyesterpolyacrylatpolyole; Polyesterpolyurethanpolyole; Polyurethanpolyacrylatpolyole, Polyurethanmodifizierte Alkydharze; Fettsäuremodifizierte Polyesterpolyurethanpolyole, Copolymerisate mit Allylethern, Propfpolymerisate aus den genannten Stoffgruppen mit z.B. unterschiedlichen Glasübergangstemperaturen, sowie Mischungen der genannten Bindemittel handeln. Bevorzugt sind Polyacrylatpolyole, Polyesterpolyole und Polyetherpolyole.

Bevorzugte OH-Zahlen, gemessen gemäß DIN 53240-2, sind 40-350 mg KOH/g Festharz für Polyester, bevorzugt 80-180 mg KOH/g Festharz, und 15-250 mg KOH/g Festharz für Polyacrylatole, bevorzugt 80-160 mg KOH/g.

Zusätzlich können die Bindemittel eine Säurezahl gemäß DIN EN ISO 3682 bis 200 mg KOH/g, bevorzugt bis 150 und besonders bevorzugt bis 100 mg KOH/g aufweisen.

Polyacrylatpolyole weisen bevorzugt ein Molekulargewicht Mₙ von mindestens 1000, besonders bevorzugt mindestens 2000 und ganz besonders bevorzugt mindestens 5000 g/mol auf. Das Molekulargewicht Mₙ kann prinzipiell nach oben unbegrenzt sein, bevorzugt bis 200.000, besonders bevorzugt bis zu 100.000, ganz besonders bevorzugt bis zu 80.000 und insbesondere bis zu 50.000 g/mol betragen.

Letztere können beispielsweise Monoester von α,β-ungesättigten Carbonsäuren, wie Acrylsäure, Methacrylsäure (in dieser Schrift kurz als "(Meth)acrylsäure" bezeichnet), mit Di- oder Polyolen, die vorzugsweise 2 bis 20 C-Atome und wenigstens zwei Hydroxygruppen aufweisen, wie Ethylenglykol, Diethylenglykol, Triethylenglykol, 1,2-Propylenglykol, 1,3-Propylenglykol, 1,1-Dimethyl-1,2-Ethandiol, Dipropylenglykol, Triethylenglykol, Tetraethylenglykol, Pentaethylenglykol, Tripropylenglykol, 1,4-Butandiol, 1,5-Pentandiol, Neopentylglykol, Hydroxypivalinsäureneopentylglykolester, 2-Ethyl-1,3-Propandiol, 2-Methyl-1,3-Propandiol, 2-Butyl-2-ethyl-1,3-Propandiol, 1,6-Hexandiol, 2-Methyl-1,5-pentandiol, 2-Ethyl-1,4-butandiol, 2-Ethyl-1,3-Hexandiol, 2,4-Diethyl-oktan-1,3-diol, 2,2-Bis(4-hydroxycyclohexyl)propan, 1,1-, 1,2-, 1,3- und 1,4-Bis(hydroxymethyl)-cyclohexan, 1,2-, 1,3- oder 1,4-Cyclohexandiol, Glycerin, Trimethylolethan, Trimethylolpropan, Trimethylolbutan, Pentaerythrit, Ditrimethylolpropan, Dipentaerythrit, Sorbit, Mannit, Diglycerol, Threit, Erythrit, Adonit (Ribit), Arabit (Lyxit), Xylit, Dulcit (Galactit), Maltit, Isomalt, Poly-THF mit einem Molgewicht zwischen 162 und 4500, bevorzugt 250 bis 2000, Poly-1,3-propandiol oder Polypropylenglykol mit einem Molgewicht zwischen 134 und 2000 oder Polyethylenglykol mit einem Molgewicht zwischen 238 und 2000 sein.

Bevorzugt sind 2-Hydroxyethylacrylat, 2-Hydroxyethylmethacrylat, 2- oder 3-Hydroxypropylacrylat, 1,4-Butandiolmonoacrylat oder 3-(Acryloyloxy)-2-hydroxypropylacrylat und besonders bevorzugt 2-Hydroxyethylacrylat und/oder 2-Hydroxyethylmethacrylat.

Die hydroxygruppentragenden Monomere werden in die Copolymerisation im Gemisch mit anderen polymerisierbaren, bevorzugt radikalisch polymerisierbaren Monomeren, eingesetzt, bevorzugt solche, welche zu mehr als 50 Gew% aus C₁-C₂₀-, bevorzugt C₁- bis C₄-Alkyl(meth)acrylat, (Meth)acrylsäure, Vinylaromaten mit bis zu 20 C-Atomen, Vinylestern von bis zu 20 C-Atomen enthaltenden Carbonsäuren, Vinylhalogeniden, nicht aromatischen Kohlenwasserstoffen mit 4 bis 8 C-Atomen und 1 oder 2 Doppelbindungen, ungesättigten Nitrilen und deren Mischungen bestehen. Besonders bevorzugt sind die Polymeren, die zu mehr als 60 Gew% aus C₁-C₁₀-Alkyl(meth)acrylaten, Styrol und dessen Derivate, Vinylimidazol oder deren Mischungen bestehen.

Darüber können die Polymere hydroxyfunktionelle Monomere entsprechend dem obigen Hydroxygruppengehalt und gegebenenfalls weitere Monomere enthalten, z.B. (Meth)acrylsäureglycidylepoxyester, ethylenisch ungesättigte Säuren, insbesondere Carbonsäuren, Säureanhydride oder Säureamide.

Weitere Polymere sind z.B. Polyesterole, wie sie durch Kondensation von Polycarbonsäuren, insbesondere Dicarbonsäuren mit Polyolen, insbesondere Diolen erhältlich sind. Um eine für die Polymerisation angemessene Funktionalität des Polyesterpolyols zu gewährleisten werden partiell auch Triole, Tetrole etc. wie auch Trisäuren, etc. eingesetzt.

Polyesterpolyole, sind z.B. aus Ullmanns Encyklopädie der technischen Chemie, 4. Auflage, Band 19, S. 62 bis 65 bekannt. Bevorzugt werden Polyesterpolyole eingesetzt, die durch Umsetzung von zweiwertigen Alkoholen mit zweiwertigen Carbonsäuren erhalten werden. Anstelle der freien Polycarbonsäuren können auch die entsprechenden Polycarbonsäureanhydride oder entsprechende Polycarbonsäureester von niederen Alkoholen oder deren Gemische zur Herstellung der Polyesterpolyole verwendet werden. Die Polycarbonsäuren können aliphatisch, cycloaliphatisch, aromatisch oder heterocyclisch sein und gegebenenfalls, z.B. durch Halogenatome, substituiert und/oder ungesättigt sein. Als Beispiele hierfür seien genannt:
Oxalsäure, Maleinsäure, Fumarsäure, Bernsteinsäure, Glutarsäure, Adipinsäure, Sebacinsäure, Dodekandisäure, o-Phthalsäure, Isophthalsäure, Terephthalsäure, Trimellithsäure, Azelainsäure, 1,4-Cyclohexandicarbonsäure oder Tetrahydrophthalsäure, Korksäure, Azelainsäure, Phthalsäureanhydrid, Tetrahydrophthalsäureanhydrid, Hexahydrophthalsäureanhydrid, Tetrachlorphthalsäureanhydrid, Endomethylentetrahydrophthalsäureanhydrid, Glutarsäureanhydrid, Maleinsäureanhydrid, dimere Fettsäuren, deren Isomere und Hydrierungsprodukte sowie veresterbare Derivate, wie Anhydride oder Dialkylester, beispielsweise C₁-C₄-Alkylester, bevorzugt Methyl-, Ethyl- oder n-Butylester, der genannten Säuren eingesetzt werden. Bevorzugt sind Dicarbonsäuren der allgemeinen Formel HOOC-(CH₂)_{y}-COOH, wobei y eine Zahl von 1 bis 20, bevorzugt eine gerade Zahl von 2 bis 20 ist, besonders bevorzugt Bernsteinsäure, Adipinsäure, Sebacinsäure und Dodecandicarbonsäure.

Als mehrwertige Alkohole kommen zur Herstellung der Polyesterole in Betracht 1,2-Propandiol, Ethylenglykol, 2,2-Dimethyl-1,2-Ethandiol, 1,3-Propandiol, 1,2-Butandiol, 1,3-Butandiol, 1,4-Butandiol, 3-Methylpentan-1,5-diol, 2-Ethylhexan-1,3-diol, 2,4-Diethyloctan-1,3-diol, 1,6-Hexandiol, Poly-THF mit einer Molmasse zwischen 162 und 4500, bevorzugt 250 bis 2000, Poly-1,3-propandiol mit einer Molmasse zwischen 134 und 1178, Poly-1,2-propandiol mit einer Molmasse zwischen 134 und 898, Polyethylenglykol mit einer Molmasse zwischen 106 und 458, Neopentylglykol, Hydroxypivalinsäureneopentylglykolester, 2-Ethyl-1,3-Propandiol, 2-Methyl-1,3-Propandiol, 2,2-Bis(4-hydroxycyclohexyl)propan, 1,1-, 1,2-, 1,3-und 1,4-Cyclohexandimethanol, 1,2-, 1,3- oder 1,4-Cyclohexandiol, Trimethylolbutan, Trimethylolpropan, Trimethylolethan, Neopentylglykol, Pentaerythrit, Glycerin, Ditrimethylolpropan, Dipentaerythrit, Sorbit, Mannit, Diglycerol, Threit, Erythrit, Adonit (Ribit), Arabit (Lyxit), Xylit, Dulcit (Galactit), Maltit oder Isomalt, die gegebenenfalls wie oben beschrieben alkoxyliert sein können.

Bevorzugt sind Alkohole der allgemeinen Formel HO-(CH₂)ₓ-OH, wobei x eine Zahl von 1 bis 20, bevorzugt eine gerade Zahl von 2 bis 20 ist. Bevorzugt sind Ethylenglycol, Butan-1,4-diol, Hexan-1,6-diol, Octan-1,8-diol und Dodecan-1,12-diol. Weiterhin bevorzugt ist Neopentylglykol.

Ferner kommen auch Polycarbonat-Diole in Betracht, wie sie z.B. durch Umsetzung von Phosgen mit einem Überschuß von den als Aufbaukomponenten für die Polyesterpolyole genannten niedermolekularen Alkohole erhalten werden können.

Geeignet sind auch Polyesterdiole auf Lacton-Basis, wobei es sich um Homo- oder Mischpolymerisate von Lactonen, bevorzugt um endständige Hydroxylgruppen aufweisende Anlagerungsprodukte von Lactonen an geeignete difunktionelle Startermoleküle handelt. Als Lactone kommen bevorzugt solche in Betracht, die sich von Verbindungen der allgemeinen Formel HO-(CH₂)_{z}-COOH ableiten, wobei z eine Zahl von 1 bis 20 ist und ein H-Atom einer Methyleneinheit auch durch einen C₁- bis C₄-Alkylrest substituiert sein kann. Beispiele sind ε-Caprolacton, β-Propiolacton, gamma-Butyrolacton und/oder Methyl-ε-caprolacton, 4-Hydroxybenzoesäure, 6-Hydroxy-2-naphthalinsäure oder Pivalolacton sowie deren Gemische. Geeignete Starterkomponenten sind z.B. die vorstehend als Aufbaukomponente für die Polyesterpolyole genannten niedermolekularen zweiwertigen Alkohole. Die entsprechenden Polymerisate des ε-Caprolactons sind besonders bevorzugt. Auch niedere Polyesterdiole oder Polyetherdiole können als Starter zur Herstellung der Lacton-Polymerisate eingesetzt sein. Anstelle der Polymerisate von Lactonen können auch die entsprechenden, chemisch äquivalenten Polykondensate der den Lactonen entsprechenden Hydroxycarbonsäuren, eingesetzt werden.

Weiterhin sind als Polymere auch Polyetherole geeignet, die durch Addition von Ethylenoxid, Propylenoxid oder Butylenoxid an H-aktive Komponenten hergestellt werden. Ebenso sind Polykondensate aus Butandiol geeignet.

Weiterhin können hydroxyfunktionelle Carbonsäuren eingesetzt werden, wie beispielsweise Dimethylolpropionsäure oder Dimethylolbutansäure.

Bei den Polymeren kann es sich natürlich auch um Verbindungen mit primären der sekundären Aminogruppen handeln.

Dazu werden Polyisocyanatzusammensetzung und Bindemittel in einem Molverhältnis von Isocyanatgruppen zu gegenüber Isocyanat reaktiven Gruppen von 0,1:1 bis 10:1, bevorzugt 0,2:1 bis 5:1, besonders bevorzugt 0,3:1 bis 3:1, ganz besonders bevorzugt 0,5:1 bis 2:1, insbesondere 0,8:1 bis 1,2:1 und speziell 0,9:1 bis 1,1:1 miteinander vermischt, wobei gegebenenfalls noch weitere lacktypische Bestandteile eingemischt werden können, und auf das Substrat aufgetragen.

Anschließend wird das Lackgemisch unter geeigneten Bedingungen ausgehärtet. Je nach Anwendung kann dies beispielsweise bei 100 bis 140 °C erfolgen, beispielsweise bei Lacken in OEM-Anwendungen, oder in einem niedrigeren Temperaturintervall von beispielsweise 20 bis 80 °C.

Dies erfordert je nach Temperatur in der Regel nicht mehr 12 Stunden, bevorzugt bis zu 8 Stunden, besonders bevorzugt bis zu 6, ganz besonders bevorzugt bis zu 4 und insbesondere bis zu 3 Stunden.

Ferner können Beschichtungsmassen 0 bis 10 Gew% mindestens eines UV Stabilisators enthalten.

Geeignete Stabilisatoren umfassen typische UV-Absorber wie Oxanilide, Triazine und Benzotriazol (letztere erhältlich als Tinuvin® -Marken der Ciba-Spezialitätenchemie) und Benzophenone.

Diese können zusätzlich 0 bis 5 Gew% geeigneten Radikalfängern, beispielsweise sterisch gehinderten Aminen wie 2,2,6,6-Tetramethylpiperidin, 2,6-Di-tert.-butylpiperidin oder deren Derivaten, z. B. Bis-(2,2,6,6-tetra-methyl-4-piperidyl)sebacinat enthalten.

Ferner können Beschichtungsmassen weiterhin 0 bis 10 Gew% weiterer lacktypischer Additive enthalten.

Als weitere lacktypische Additive können beispielsweise Antioxidantien, Aktivatoren (Beschleuniger), Füllmittel, Pigmente, Farbstoffe, antistatische Agentien, Flammschutzmittel, Verdicker, thixotrope Agentien, oberflächenaktive Agentien, Viskositätsmodifikatoren, Plastifizierer oder Chelatbildner verwendet werden.

Als Verdicker kommen neben radikalisch (co)polymerisierten (Co)Polymerisaten, übliche organische und anorganische Verdicker wie Hydroxymethylcellulose oder Bentonit in Betracht.

Als Chelatbildner können z.B. Ethylendiaminoessigsäure und deren Salze sowie β-Diketone verwendet werden.

Geeignete Füllstoffe umfassen Silikate, z. B. durch Hydrolyse von Siliciumtetrachlorid erhältliche Silikate wie Aerosil® der Fa. Degussa, Kieselerde, Talkum, Aluminiumsilikate, Magnesiumsilikate, Calciumcarbonate etc.

Die Beschichtung der Substrate erfolgt nach üblichen, dem Fachmann bekannten Verfahren, wobei man wenigstens eine Beschichtungsmasse auf das zu beschichtende Substrat in der gewünschten Stärke aufbringt und die gegebenenfalls enthaltenen flüchtigen Bestandteile der Beschichtungsmasse, gegebenenfalls unter Erhitzen, entfernt. Dieser Vorgang kann gewünschten falls ein- oder mehrfach wiederholt werden. Das Aufbringen auf das Substrat kann in bekannter Weise, z. B. durch Spritzen, Spachteln, Rakeln, Bürsten, Rollen, Walzen, Gießen, Laminieren, Hinterspritzen oder Coextrudieren erfolgen.

Die Dicke einer solchen zu härtenden Schicht kann von 0,1 µm bis mehrere mm betragen, bevorzugt von 1 bis 2.000 µm, besonders bevorzugt 5 bis 200 µm, ganz besonders bevorzugt von 5 bis 60 µm (bezogen auf den Lack im Zustand in dem das Lösungsmittel aus dem Lack entfernt ist).

Weiterhin sind auch Substrate, beschichtet mit einem Lack, der die erfindungsgemäßen allophanatgruppenhaltigen Polyisocyanate enthält, Gegenstand der vorliegenden Erfindung.

Besonders geeignet sind solche Polyurethanlacke für Anwendungen, in denen eine besonders hohe Applikationssicherheit, Außenwitterungsbeständigkeit, Optik, Lösemittel-, Chemikalien- und Wasserfestigkeit gefordert werden.

Die erhaltenen zweikomponentigen Beschichtungsmassen und Lackformulierungen eignen sich prinzipiell zum Beschichten von Substraten wie Holz, Holzfurnier, Papier, Pappe, Karton, Textil, Folie, Leder, Vlies, Kunststoffoberflächen, Glas, Keramik, mineralischen Baustoffen, wie Zement-Formsteine und Faserzementplatten oder Metallen, die jeweils optional vorbeschichtet bzw. vorbehandelt sein können. Besonders bevorzugt sind sie jedoch zum Beschichten von Kunststoffoberflächen und metallischen Substraten geeignet.

Bevorzugt werden diese Beschichtungsmassen als Klar-, Basis- und Decklacke(n), Primern und Füller eingesetzt, besonders eignen sie sich durch ihre hohe Kratzfestigkeit als Decklack, bevorzugt als Klarlack, insbesondere in Beschichtungen auf (Groß-)Fahrzeugen und Flugzeugen und in Automobillacken als OEM und refinish-Anwendung.

Es ist ein Vorteil der erfindungsgemäßen allophanatgruppenhaltigen Polyisocyanate, daß sie in Klarlacken eine hohe Härte bei gleichzeitig guter Elastizität ergeben. Zudem ergeben die erfindungsgemäßen Produkte eine hohe Funktionalität.

### Beispiele

### Beispiel 1

800g monomeres Isophorondiisocyanat (3,6 mol) wurden mit 47,9 g (0,18 mol) propoxyliertem Glycerin, das je Hydroxygruppe im Mittel eine Propylenoxidgruppe trägt, versetzt und auf 80°C erwärmt. Zu der klaren Lösung wurden 0,2 g Zinkacetylacetonat zugegeben. Die Mischung wurde ca. fünf Stunden bei 120°C gehalten. Der NCO Gehalt betrug 30,6 %. Zu der Mischung wurden 0,2 ml Diethylhexylphosphat gegeben. In einem Dünnschichtverdampfer wurde bei 165°C Außentemperatur und 4,1 mbar nicht umgesetztes Monomer entfernt.

Der NCO Gehalt des festen Produktes betrug 13,2 %. Eine 70%ige Lösung des Produktes in Butylacetat hatte einen NCO Gehalt von 10,4 % und eine Viskosität von 2340 mPas.

### Vergleichsbeispiel 1

800 g (4,76 mol) 1,6-Hexamethylendiisocyanat wurden mit 63,3 g (9,24 mol) des gleichen propoxylierten Glycerin wie in Beispiel 1 versetzt und auf 80°C erwärmt. Zu der klaren Lösung wurden 0,2 g Zinkacetylacetonat zugegeben. Die Mischung wurde auf 120°C erwärmt.

Nach einer Stunde war der Kolbeninhalt vernetzt.

### Anwendungsbeispiele:

Das Polyisocyanat aus Beispiel 1, sowie ein Vergleichspolyisocyanat (Mischung eines handelsüblichen Isocyanurats auf Basis 1,6-Hexamethylendiisocyanat (NCO-Gehalt etwa 22,0 Gew%, Viskosität gemäß DIN EN ISO 3219 bei 23 °C und 1000 s⁻¹ ca. 3300 mPas, Basonat® HI 100 der BASF AG, Ludwigshafen) und eines handelsüblichen Isocyanurats auf Basis Isophorondiisocyanat (NCO-Gehalt etwa 12,0 Gew%, 70%ig in Butylacetat, Viskosität gemäß DIN EN ISO 3219 bei 23 °C und 1000 s⁻¹ ca. 700 mPas, Basonat® IT 170 B der BASF AG, Ludwigshafen)) im stöichiometrischen Verhältnis 7:3), wurden mit einem hydroxyfunktionellen Polyacrylatharz (Macrynal® SM 600, Fa. Cytec; Festgehalt = 60%; OH-Zahl = 100 mg KOH/g) entsprechend einen stöichiometrischen NCO/OH-Verhältniss von 1:1 gemischt und mit Butylacetat auf einer Applikationsviskosität von 20 s (DIN 53 211 Becher 4 mm Auslaufsdüse) eingestellt.
Mit einem Ziehrahmen wurden auf Metallbleche Beschichtungen mit einer Nassfilmdicke von 200 µm aufgetragen. Die so enthaltenen Klarlacken wurden nach 10 Minuten Ablüftungszeit bei 80°C und 130°C jeweils 30 Minuten gehärtet, und Pendelhärte und Erichsentiefe der Lacke gemessen. Die Untersuchungen der Lackeigenschaften erfolgten nach 24 Stunden Lagerung der lackierten Bleche in einem Klimaraum bei 23 °C und 50 % relativer Luftfeuchte.

Die Ermittlung der Erichsen-Tiefung erfolgte analog DIN 53156. Hohe Werte bedeuten hohe Flexibilität.

Die Ermittlung der Pendelhärte erfolgte analog DIN 53157, hohe Werte bedeuten hohe Härte.

| | Pendelhärte 80°C | Erichsen Tiefe 80°C |
|---|---|---|
| Vergleichspolyisocyanat | 118 | > 9,0 |
| Beispiel 1 | 135 | > 9,0 |

| | Pendelhärte 130°C | Erichsen Tiefe 130°C |
|---|---|---|
| Vergleichspolyisocyanat | 128 | > 9,0 |
| Beispiel 1 | 140 | 8,5 |

Die Versuche zeigen, daß die erfindungsgemäß Polyisocyanate eine vergleichbare Elastizität wie die Vergleichspolyisocyanatmischung zeigen bei verbesserter Härte.

## Patentansprüche

1. Allophanatgruppenhaltige Polyisocyanate der Formel (I) 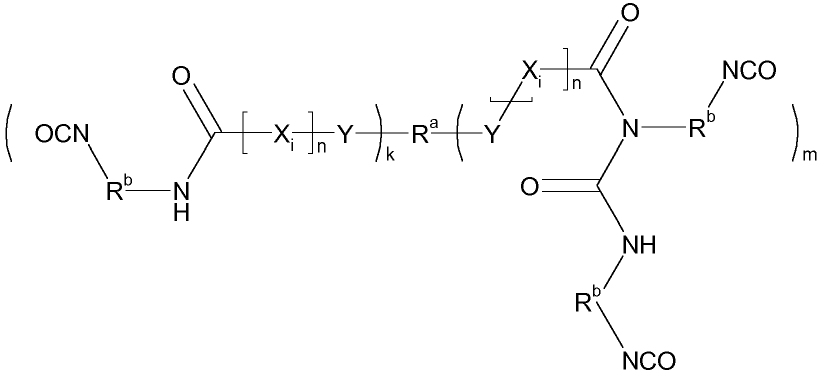 worin
R^{a} einen (k+m)-wertigen Rest, bevorzugt einen organischen Rest,
k 0 oder eine positive Zahl,
m ein positive Zahl,
mit der Maßgabe, daß (k+m) einen Wert von 3 bis 4 einnimmt,
Y für ein Sauerstoff- oder Stickstoffatom,
Xᵢ für jedes i von 1 bis n unabhängig voneinander ausgewählt ist aus der Gruppe bestehend aus -CH₂-CH₂-O-, -CH₂-CH(CH₃)-O-, -CH(CH₃)-CH₂-O-,-CH₂-C(CH₃)₂-O-, -C(CH₃)₂-CH₂-O-, -CH₂-CHVin-O-, -CHVin-CH₂-O-, -CH₂-CHPh-O- und -CHPh-CH₂-O-,
worin Ph für Phenyl und Vin für Vinyl steht,
n für jedes k und jedes m unabhängig voneinander 0 oder eine positive Zahl bedeutet, und
R^{b} für jedes k und jedes m unabhängig voneinander einen Rest 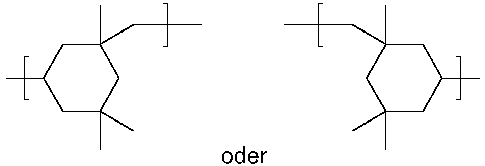 bedeutet.

2. Allophanatgruppenhaltige Polyisocyanate gemäß Anspruch 1, **dadurch gekennzeichnet, daß** der zugrundeliegende Alkohol R^{a}-(-Y-H)₍ₖ₊ₘ₎, worin Y ein Sauerstoffatom darstellt, ausgewählt ist aus der Gruppe bestehend aus Trimethylolbutan, Trimethylolpropan, Trimethylolethan, Pentaerythrit, Glycerin, Dipentaerythrit, Diglycerol, Threit and Erythrit.

3. Allophanatgruppenhaltige Polyisocyanate gemäß Anspruch 2, **dadurch gekennzeichnet, daß** Xᵢ ausgewählt ist aus der Gruppe bestehend aus -CH₂-CH₂-O-,-CH₂-CH(CH₃)-O- und -CH(CH₃)-CH₂-O-.

4. Allophanatgruppenhaltige Polyisocyanate gemäß Anspruch 1, **dadurch gekennzeichnet, daß** der zugrundeliegende Alkohol R^{a}-(-Y-[-Xᵢ]ₙ-H)₍ₖ₊ₘ₎, worin Y ein Stickstoffatom darstellt, ausgewählt ist aus der Gruppe bestehend aus Triethanolamin, Tripropanolamin und 1,3,5,Tris-(2-hydroxyethyl)cyanursäure.

5. Allophanatgruppenhaltige Polyisocyanate gemäß einem der vorstehenden Ansprüche, **dadurch gekennzeichnet, daß** der NCO-Gehalt (berechnet als NCO mit eine Molgewicht von 42 g/mol) mehr als 5 Gew% und bis zu 17 Gew% beträgt.

6. Zweikomponentige Polyurethanlacke, enthaltend allophanatgruppenhaltige Polyisocyanate gemäß einem der vorstehenden Ansprüche, gegebenenfalls weitere Polyisocyanate und mindestens eine Komponente, die gegenüber Isocyanat reaktive Gruppen enthält.

7. Verwendung von allophanatgruppenhaltigen Polyisocyanaten gemäß einem der Ansprüche 1 bis 5 in Klarlacken.

8. Verwendung von allophanatgruppenhaltigen Polyisocyanaten gemäß einem der Ansprüche 1 bis 5 in Beschichtungsmassen für (Groß-)Fahrzeuge, Flugzeuge, in Automobillacken als OEM und refinish-Anwendung.

9. Verfahren zur Herstellung von allophanatgruppenhaltigen Polyisocyanaten gemäß einem der Ansprüche 1 bis 5, **dadurch gekennzeichnet, daß** man Isophorondiisocyanat in mindestens doppelt äquimolarer Stöchiometrie bezogen auf die Hydroxygruppen im Alkohol mit einem Alkohol vermischt und in Gegenwart mindestens eines Katalysators bei einer Temperatur von mindestens 80°C miteinander umsetzt.

10. Verfahren nach Anspruch 9, **dadurch gekennzeichnet, daß** der Katalysator ausgewählt ist aus der Gruppe bestehend aus Zinksalzen, Cäsiumsalzen, Wismutsalzen und Zinnverbindungen.

## Claims

1. An allophanate-group-containing polyisocyanate of the formula (I) 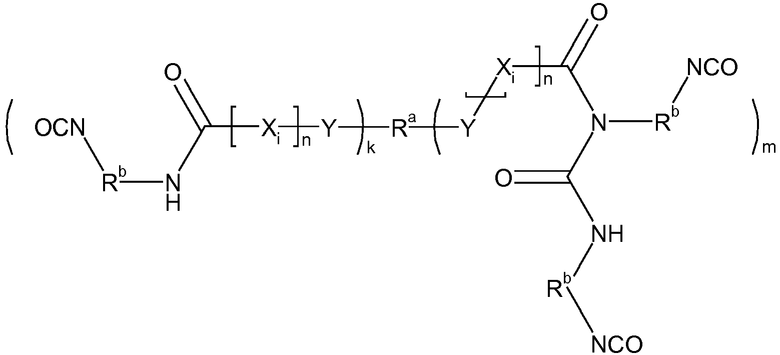 in which
R^{a} is a (k+m)-valent radical, preferably an organic radical,
k is 0 or a positive number,
m is a positive number,
with the proviso that (k + m) takes on a value of 3 to 4,
Y is an oxygen or a nitrogen atom,
Xᵢ for each i from 1 to n independently of one another is selected from the group consisting of -CH₂-CH₂-O-, -CH₂-CH(CH₃)-O-, -CH(CH₃)-CH₂-O-, -CH₂-C(CH₃)₂-O-, -C(CH₃)₂-CH₂-O-, -CH₂-CHVin-O-, -CHVin-CH₂-O-, -CH₂-CHPh-O- and -CHPh-CH₂-O-,
in which Ph is phenyl and Vin is vinyl,
n for each k and each m independently of one another is 0 or a positive number, and
R^{b} for each k and each m independently of one another is a radical

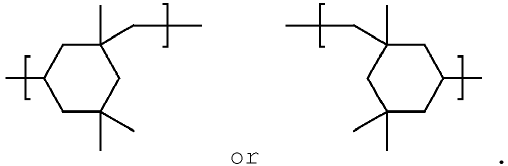

2. The allophanate-group-containing polyisocyanate according to claim 1, wherein the parent alcohol R^{a}-(-Y-H)₍ₖ₊ₘ₎, in which Y is an oxygen atom, is selected from the group consisting of trimethylolbutane, trimethylolpropane, trimethylolethane, pentaerythritol, glycerol, dipentaerythritol, diglycerol, threitol and erythritol.

3. The allophanate-group-containing polyisocyanate according to claim 2, wherein Xᵢ is selected from the group consisting of -CH₂-CH₂-O-, -CH₂-CH(CH₃)-O-, and -CH(CH₃)-CH₂-O-.

4. The allophanate-group-containing polyisocyanate according to claim 1, wherein the parent alcohol R^{a}-(-Y-[-Xᵢ]ₙ-H)₍ₖ₊ₘ₎, in which Y is a nitrogen atom, is selected from the group consisting of triethanolamine, tripropanolamine, and 1,3,5-tris(2-hydroxyethyl)cyanuric acid.

5. The allophanate-group-containing polyisocyanate according to any of the preceding claims, wherein the NCO content (calculated as NCO with a molar weight of 42 g/mol) is more than 5% by weight and up to 17% by weight.

6. A two-component polyurethane coating material comprising allophanate-group-containing polyisocyanates according to any of the preceding claims, further polyisocyanates if desired, and at least one component which comprises isocyanate-reactive groups.

7. The use of an allophanate-group-containing polyisocyanate according to any of claims 1 to 5 in clearcoat materials.

8. The use of an allophanate-group-containing polyisocyanate according to any of claims 1 to 5 in coating compositions for (large) vehicles, aircraft, and in automobile finishes as OEM and refinish.

9. A process for preparing an allophanate-group-containing polyisocyanate according to any of claims 1 to 5, which comprises mixing isophorone diisocyanate with an alcohol, in at least twice-equimolar stoichiometry relative to the hydroxyl groups in the alcohol, and reacting them together in the presence of at least one catalyst at a temperature of at least 80°C.

10. The process according to claim 9, wherein the catalyst is selected from the group consisting of zinc salts, cesium salts, bismuth salts, and tin compounds.

## Revendications

1. Polyisocyanates contenant des groupes allophanate de la formule (I) : 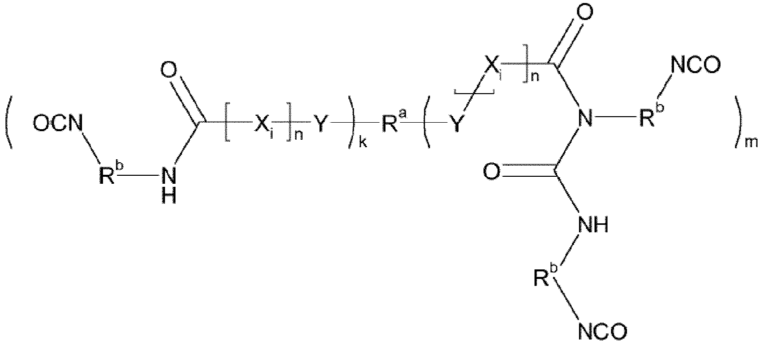 dans laquelle
R^{a} signifie un radical (k+m)-valent, de préférence un radical organique,
k signifie 0 ou un nombre positif,
m signifie un nombre positif,
à condition que (k+m) prenne une valeur de 3 à 4,
Y signifie un atome d'oxygène ou d'azote,
les Xᵢ sont choisis indépendamment les uns des autres pour chaque i de 1 à n dans le groupe constitué par-CH₂-CH₂-O-, -CH₂-CH(CH₃)-O-, -CH(CH₃)-CH₂-O-, -CH₂-C(CH₃)₂-O-, -C(CH₃)₂-CH₂-O-, -CH₂-CHVin-O-, -CHVin-CH₂-O-, -CH₂-CHPh-O- et -CHPh-CH₂-O-,
Ph représentant phényle et Vin représentant vinyle,
les n signifient indépendamment les uns des autres pour chaque k et chaque m 0 ou un nombre positif, et
les R^{b} signifient indépendamment les uns des autres pour chaque k et chaque m un radical

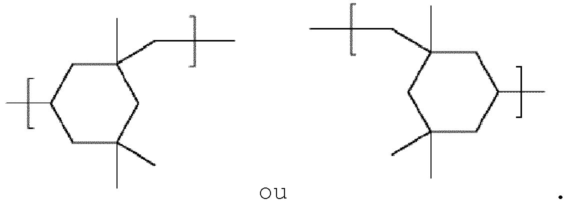

2. Polyisocyanates contenant des groupes allophanate selon la revendication 1, **caractérisés en ce que** l'alcool sous-jacent R^{a}-(-Y-H)₍ₖ₊ₘ₎ dans lequel Y représente un atome d'oxygène est choisi dans le groupe constitué par le triméthylolbutane, le triméthylolpropane, le triméthyloléthane, le pentaérythritol, la glycérine, le dipentaérythritol, le diglycérol, le thréitol et l'érythritol.

3. Polyisocyanates contenant des groupes allophanate selon la revendication 2, **caractérisés en ce que** Xᵢ est choisi dans le groupe constitué par -CH₂-CH₂-O-, -CH₂-CH(CH₃)-O- et -CH(CH₃)-CH₂-O-.

4. Polyisocyanates contenant des groupes allophanate selon la revendication 1, **caractérisés en ce que** l'alcool sous-jacent R^{a}-(-Y-[-Xᵢ]ₙ-H)₍ₖ₊ₘ₎ dans lequel Y représente un atome d'azote est choisi dans le groupe constitué par la triéthanolamine, la tripropanolamine et l'acide 1,3,5-tris-(2-hydroxyéthyl)cyanurique.

5. Polyisocyanates contenant des groupes allophanate selon l'une quelconque des revendications précédentes, **caractérisés en ce que** la teneur en NCO (calculée en tant que NCO ayant un poids moléculaire de 42 g/mol) est de plus de 5 % en poids et de jusqu'à 17 % en poids.

6. Vernis bicomposant à base de polyuréthane, contenant des polyisocyanates contenant des groupes allophanate selon l'une quelconque des revendications précédentes, éventuellement des polyisocyanates supplémentaires et au moins un composant qui contient des groupes réactifs avec les isocyanates.

7. Utilisation de polyisocyanates contenant des groupes allophanate selon l'une quelconque des revendications 1 à 5 dans des vernis transparents.

8. Utilisation de polyisocyanates contenant des groupes allophanate selon l'une quelconque des revendications 1 à 5 dans des matériaux de revêtement pour véhicules (de grande taille), aéronefs, dans des vernis automobiles en tant qu'application d'OEM et de réfection.

9. Procédé de fabrication de polyisocyanates contenant des groupes allophanate selon l'une quelconque des revendications 1 à 5, **caractérisé en ce que** du diisocyanate d'isophorone est mélangé avec un alcool en une stœchiométrie au moins deux fois équimolaire par rapport au groupes hydroxy dans l'alcool, et mis en réaction en présence d'au moins un catalyseur à une température d'au moins 80 °C.

10. Procédé selon la revendication 9, **caractérisé en ce que** le catalyseur est choisi dans le groupe constitué par les sels de zinc, les sels de césium, les sels de bismuth et les composés d'étain.
